(19) Europäisches Patentamt
European Patent Office
Office européen des brevets

(11) Veröffentlichungsnummer: **0 000 026**
**A1**

(12) # EUROPÄISCHE PATENTANMELDUNG

(21) Anmeldenummer: **78100041.9**

(51) Int. Cl.$^2$: **C 07 C 85/08**

(22) Anmeldetag: **01.06.78**

(30) Priorität: **06.06.77 DE 2725669**

(43) Veröffentlichungstag der Anmeldung:
**20.12.78 Patentblatt 78/1**

(84) Benannte Vertragsstaaten:
**BE DE FR**

(71) Anmelder: **BASF Aktiengesellschaft,**
**Carl-Bosch-Strasse 38,**
**D-6700 Ludwigshafen (DE)**

(72) Erfinder: **Merkel, Karl, Dr.,**
**Sperlinggasse 15,**
**D-6700 Ludwigshafen (DE)**

(72) Erfinder: **Toussaint, Herbert, Dr.,**
**Knietschstrasse 21,**
**D-6710 Frankenthal (DE)**

(72) Erfinder: **Mueller, Herbert, Dr.,**
**Carostrasse 53,**
**D-6710 Frankenthal (DE)**

(72) Erfinder: **Hoffmann, Herwig, Dr.,**
**Knietschstrasse 21,**
**D-6710 Frankenthal (DE)**

(72) Erfinder: **Hupfer, Leopold, Dr.,**
**Waltershöhe 3,**
**D-6701 Friedelsheim (DE)**

(72) Erfinder: **Mercker, Hans Jochen, Dr.,**
**Schwabenstrasse 14,**
**D-6800 Mannheim 61 (DE)**

(54) **Verfahren zur Herstellung von sekundären Aminen.**

(57) Verfahren zur Herstellung von sekundären Aminen durch N-Alkylierung von primären Aminen mittels Aldehyden unter hydrierenden Bedingungen in Gegenwart einer quaternären Ammoniumbase.

EP 0 000 026 A1

## Verfahren zur Herstellung von sekundären Aminen

Es sind zahlreiche Methoden zur Herstellung von sekundären Aminen bekannt, z.B. durch Umsetzung von primären Aminen oder Ammoniak bei erhöhten Temperaturen, mit Alkylhalogeniden, Alkoholen, Ketonen oder Olefinen. Eine Übersicht über diese Methoden ist in dem Werk von Houben-Weyl, "Methoden der organischen Chemie", 4. Aufl., Band 11/1, enthalten. Nach diesen Methoden erhält man jedoch im allgemeinen Mischungen aus primären, sekundären und tertiären Aminen. Die Isolierung der sekundären Amine aus den Gemischen, z.B. durch Destillation, erfordert einen hohen Destillationsaufwand.

Im technischen Maßstab werden sekundäre Amine vorzugsweise durch Umsetzung von Aldehyden mit primären Aminen unter hydrierenden Bedingungen in Gegenwart eines Hydrierkatalysators und einer Base, insbesondere NaOH oder KOH hergestellt.

Dieses Verfahren hat jedoch Nachteile:

Die Ausbeuten an sekundären Aminen betragen höchstens 80 bis 85 % der theoretischen möglichen Ausbeute, so daß die Reinigung der rohen Amine von Nebenprodukten aufwendig ist.

Mu/BL

Die alkalischen Katalysatoren bilden mit den Aminen oft unlösliche, feste bis halbfeste Produkte unbekannter Konstitution. Diese Stoffe führen vor allem bei fortlaufendem (kontinuerlichen) Betrieb zu Störungen, da sie sich in den Leitungen und Ventilen im Bereich der Hydrier-Reaktoren und außerdem auf der Oberfläche der Hydrier-Katalysatoren ablagern, so daß deren Aktivität und damit die Ausbeute stetig abnimmt.

Es ist eine Aufgabe der Erfindung, das vorstehend beschriebene Verfahren hinsichtlich der möglichen Ausbeute und der Standzeit der Katalysatoren zu verbessern. Es wurde gefunden, daß die Ausbeuten an sekundären Aminen auf über 95 % der berechneten gesteigert, Ablagerungen und Feststoffen in den Leitungen vermieden und die Lebensdauer der Hydrier-Katalysatoren erheblich gesteigert werden können, wenn man anstelle einer anorganischen Base eine quaternäre Ammoniumbase verwendet.

Quaternäre Ammoniumbasen, die durch die allgemeine Formel (I)

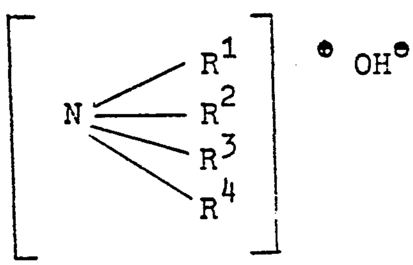

dargestellt werden können, wobei die Reste $R^1$ bis $R^4$ je eine gleiche oder verschiedene Alkyl-, Cycloalkyl-, Aryl-, Aralkylgruppe, die ihrerseits funktionelle Gruppen enthalten können bedeuten, sind im allgemeinen gut bekannte Verbindungen. Sie können allgemein durch vollständige Alkylierung von Ammoniak oder Aminen bzw. Ammoniumsalzen mit den bekannten Alkylierungsmitteln erhalten werden; falls Salze der Ammoniumbasen mit Säureanionen vorliegen, können diese

in bekannter Weise unter Freisetzung der Base gespalten werden. Ersichtlich ist hierbei die Wahl der Substituenten $R^1$ bis $R^2$ nicht entscheidend; die Reste $R^1$ bis $R^4$ können z.B. jeweils 1 bis 20 Kohlenstoffatome aufweisen.

Unmittelbar werden quaternäre Ammoniumbasen erhalten, wenn man Ammoniak oder Amine mit Alkylenoxiden als Alkylierungsmittel umsetzt, wobei stufenweise die Mono-, Di- und Trialkanolamine und schließlich in Gegenwart von etwas Wasser Tetrahydroxyalkylammonium-hydroxide gebildet werden. Bevorzugt sind Umsetzungsprodukte von Ammoniak mit Äthylen- oder Propylenoxid.

Dieser Sachverhalt, der an sich wenig bekannt ist, führt dazu, daß bei der Herstellung der Alkanolamine ein stark basisches Nebenprodukt gebildet wird, das nach dem Abdestillieren der Amine hinterbleibt. Die Untersuchung hat gezeigt, daß es sich offenbar um quaternäre Ammoniumbasen der o.a. Struktur handelt, jedoch soll die Erfindung im Kern so verstanden werden, daß der Destillationsrückstand einer solchen Synthese der Alkanolamine ein besonders bevorzugter Katalysator ist, der leicht zugänglich und bei größerem Bedarf auch absichtlich, d.h. nicht nur als Nebenprodukt hergestellt werden kann.

Der Vorschlag, diesen Destillationsrückstand anstelle einer anorganischen Base zu verwenden, ist schon in der DT-OS 21 21 325 beschrieben. Das Verfahren dieser Beschreibung unterscheidet sich jedoch von dem vorliegenden Verfahren grundsätzlich dadurch, daß nicht unter relativ scharfen, hydrierenden Bedingungen gearbeitet wird. Die Beständigkeit und Anwendbarkeit einer quaternären Base unter hydrierenden Bedingungen war nicht ohne weiteres zu erwarten, da es bekannt ist, daß unter diesen Bedingungen empfindliche Amine entalkyliert werden, u.U. bis zum Ammoniak. Auch kann aus

der Hydroxyalkylgruppe Wasser abgespalten werden, wobei Verbindungen entstehen, die verharzen können.

Das Verfahren der Erfindung kann mit den üblichen Hydrierkatalysatoren durchgeführt werden, die z.B. bei Houben-Weyl a.a.o. S. 602 beschrieben sind. Es sind dies z.B. Metalle, wie Palladium, Platin, Eisen, Rhodium, Rhenium, Ruthenium oder deren Verbindungen und Gemische in Form feinverteilter Suspensionen oder auf Trägern für Festbettkatalysatoren; ferner Molybdän- und Wolframkatalysatoren und mit besonderem Vorteil Kobalt- und Nickelkatalysatoren. Kobalt- und Nickelkatalysatoren können ebenfalls in trägerfreier Form, d.h. als Raney-Kobalt- bzw. -Nickel oder sogenannte Sinter- bzw. Vollkatalysatoren oder auf Trägern aufgebracht sein. Sie können Beimengen der obengenannten Metalle bzw. Metallverbindungen enthalten und ferner auch Eisen, Kupfer, Mangan, Chrom, Molybdän, Silber und ferner Alkali- oder Erdalkali-Metalle oder deren Verbindungen, weiter durch Aluminium, Vanadium oder Bor bzw. deren Sauerstoffverbindungen oder durch Phosphorsäure modifiziert sein. Als Träger können insbesondere Aluminiumoxide, Silikate, Bimsstein, Ton, Kohle usw. dienen.

Das Verfahren kann absatzweise, unter Ausnützung der Vorteile der Erfindung aber besonders vorteilhaft fortlaufend durchgeführt werden. Bei der fortlaufenden (kontinuierlichen) Methode verwendet man zweckmäßig senkrecht stehende zylindrische, druckfeste Rohre, die mit einem Festbettkatalysator in Form von Pillen oder Strängen gefüllt sind. Die Ausgangsstoffe (Aldehyd, Amin, Wasserstoff) können von oben nach unten (z.B. nach der Rieselmethode), von unten nach oben (Sumpfmethode) oder im Gegenstrom durch den Reaktor geführt werden. Ebenso ist mit suspendierten Katalysatoren ein kontinuierlicher Betrieb möglich, wobei die Sinkgeschwindigkeit der Katalysatorenpartikel zu ihrer Trennung

vom Reaktionsprodukt benützt wird.

Die Umsetzung wird im allgemeinen bei bis zu 500 bar, zweckmäßig bei 50 bis 250 bar und bei 30 bis 300°C, vorzugsweise bei 70 bis 130°C, durchgeführt. Die erfindungsgemäß zugesetzte Base wird mit dem Reaktionsgemisch ausgetragen und kann nach Abtrennung der gebildeten Amine zurückgeführt werden.

Beispiel 1

Absatzweise Herstellung von Methyl-butyl-amin nach der Gleichung

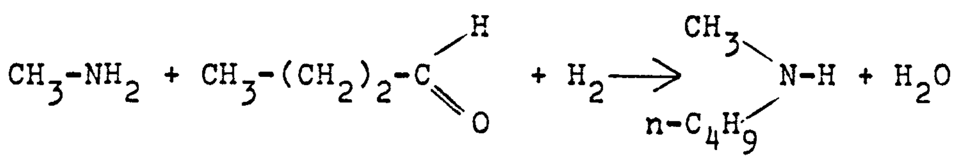

Apparatur: Druckautoklav aus Stahl von 3 000 l Inhalt, mit einer Rührwelle zur Homogenisierung der Reaktanten und üblichen Anbauten und Hilfsmitteln.

Ablauf der Reaktion: Es werden eingefüllt: 451 kg wäßrige 76,0 prozentige Methylamin-Lösung (11 kg-Mol), 792 kg n-Butyraldehyd (10,98 kg-Mol), 10 kg Tetrahydroxyäthyl-ammoniumhydroxid, 40 kg Raney-Nickel.

Die Umsetzung wird bei einem Wasserstoffdruck von 120 bar und einer Reaktionstemperatur von 105 bis 115°C während 6 Stunden durchgeführt.

Das Reaktionsgemisch wird filtriert. Aus dem Filtrat wird das Wasser durch Destillation abgetrennt. Der Destillationsrückstand (950 kg) besteht zu 95,5 % aus Methyl-n-butylamin. Als Nebenprodukt werden Dimethyl-, Mono-butyl-,

Di-butyl-, Di-butyl-methyl-amin und n-Butanol gefunden. Die Rohausbeute an Methyl-n-butyl-amin beträgt 94,8 % der berechneten. Das rohe Amin wird durch nochmalige Destillation gereinigt. Man erhält 916 kg Methyl-n-butylamin mit einer Reinheit von 98,9 %.

Vergleichsversuch zu Beispiel 1

Man verfährt nach den Einzelangaben des Beispiels 1, anstelle der quaternären Base werden jedoch 10 kg NaOH eingesetzt.

Ergebnis: Nach Filtration und Abtrennen des Wassers erhält man 948 kg rohes, wasserfreies Methyl-n-butylamin mit einer Reinheit von 83,5 %, d.h. 16,5 % Nebenprodukte. Die Ausbeute beträgt 82,7 % der berechneten.

Das rohe Amin wird durch abermalige Destillation gereinigt. Man erhält 775 kg Methyl-n-butyl-amin mit einer Reinheit von 98,0 %.

Es treten außerdem mehrfach Betriebsstörungen dadurch auf, daß die Ablaufrohre und Durchlaufventile für das Rohamin vom Reaktionsbehälter zu den Vorratsbehältern durch feste Ablagerungen verstopft waren.

Beispiel 2

Fortlaufende Herstellung von Äthyl-n-butylamin nach der Gleichung

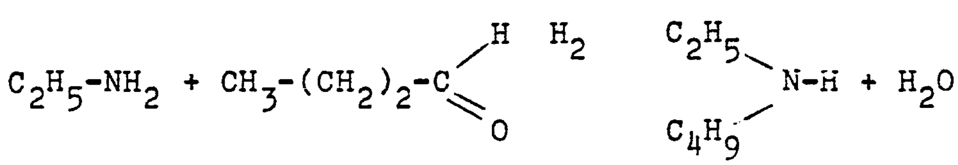

Apparatur: zylindrisch senkrecht stehendes Hochdruck-Reaktionsrohr mit einer Länge von 8 m (Inhalt = 100 l) gefüllt mit 95 l eines Festbettkatalysators aus trägerfreiem Nickel in Form von Strängen; Zulauf für Amin und Aldehyd getrennt am Kopf des Reaktors; Druckanschluß für Wasserstoff ebenfalls am Kopf des Reaktors. Gewinnung des Reaktionsgemisches am Boden des Reaktors über einen Flüssigkeitsdruckabscheider.

In den Kopf des Reaktors werden stündlich 11,7 kg wasserfreies Äthylamin mit einem Gehalt von 3,2 % Monomethyltrihydroxyäthylammoniumhydroxid als Katalysator und 18 kg n-Butyraldehyd eingepumpt. Die maximale Temperatur im Reaktor wird auf 100 bis 110°C, der Wasserstoffdruck auf 200 bar eingestellt.

Es laufen stündlich 29,8 kg eines Gemisches ab, das aus rohem Äthyl-n-butylamin und Wasser besteht. Nach Abtrennen des Wassers erhält man 25,2 kg rohes Äthyl-n-butylamin mit einem Gehalt von 94,8 % Äthyl-n-butylamin (Ausbeute 94 % der berechneten).

Ferner werden 2,7 % n-Butanol und insgesamt 2,5 % andere Nebenprodukte (Diäthylamin, Diäthyl-n-butylamin, Di-butyläthyl-amin) und unbekannte gefunden. Nach 4 Wochen durchgehender Betriebsdauer des Reaktors wird noch die gleiche Menge Äthyl-n-butylamin, nach 8 Wochen Laufzeit noch stündlich 25,0 kg rohes Äthyl-n-butylamin mit einem Gehalt von 92,9 % Äthyl-n-butylamin erhalten.

Vergleichsversuch zu Beispiel 2

Es wird anstelle der organischen Base die gleiche Gewichtsmenge NaOH eingesetzt, ansonsten genau nach Beispiel 2 verfahren. Diese Betriebsweise war bis zum Gelingen der Erfindung die übliche.

Nach Abtrennen des Wassers und fester Anteile erhält man stündlich 25 kg rohes Äthyl-n-butylamin mit einem Gehalt von 86,1 % Äthyl-n-butylamin, entpsrechend 86 % Ausbeute. Als Nebenprodukt werden 5,0 % n-Butanol und 5,95 % andere Nebenprodukte erhalten.

Ein annähernd störungsfreier durchgehender Betrieb des Reaktors war jeweils nur kurz möglich. In Abständen von 1 bis 2 Tagen mußten die Ablaufleitungen und Ventile, die von dem rohen Reaktionsgemisch passiert werden, von festen und halbfesten Ablagerungen gereinigt werden.

Nach 14 Tagen Betriebsdauer des Hydrierreaktors ist die Aktivität des Katalysators soweit erschöpft, daß dieser erneuert werden muß.

Aus 100 kg rohem, 86,1 %igem Äthyl-n-butylamin gewinnt man durch fraktionierte Destillation nur 82,5 kg Äthyl-n-butylamin mit einer Reinheit von 98,0 %.

## Patentansprüche

1. Verfahren zur Herstellung von sekundären Aminen durch Umsetzung von Aldehyden mit primären Aminen unter hydrierenden Bedingungen in Gegenwart eines Hydrierkatalysators und einer Base (Nickel und/oder Kobalt), dadurch gekennzeichnet, daß die Umsetzung in Gegenwart einer quaternären Ammoniumbase durchgeführt wird.

2. Verfahren nach Anspruch 1, dadurch gekennzeichnet, daß als quaternäre Ammoniumbase eine Verbindung der allgemeinen Formel (I)

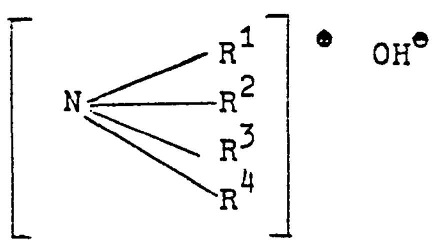

verwendet wird, wobei die Reste $R^1$ bis $R^4$ je eine gleiche oder verschiedene Alkyl-, Cycloalkyl-, Aryl-, Aralkylgruppe, die ihrerseits funktionelle Gruppen enthalten kann, bedeutet.

3. Verfahren nach Anspruch 2, dadurch gekennzeichnet, daß man eine quaternäre Base der allgemeinen Formel (I) verwendet, in der $R^1$ bis $R^4$ wenigstens einmal die Hydroxyäthyl- oder Hydroxypropylgruppe und im übrigen eine Alkylgruppe bezeichnet.

4. Verfahren nach Anspruch 3, dadurch gekennzeichnet, daß man als Base das vollständige Umsetzungsprodukt von Ammoniak oder einem primären oder sekundären Alkylamin mit Äthylenoxid verwendet, wie es erhalten wird, wenn man Ammoniak, ein primäres oder sekundäres Alkylamin mit wenigstens 4 bzw. 3 bzw. 2 Mol Äthylenoxid derart um-

setzt, daß nach dem Abdestillieren der gebildeten Alkanolamine aus dem Umsetzungsprodukt ein basischer Rückstand verbleibt, der nicht unzersetzt destillierbar ist.

5. Verfahren nach Anspruch 1, dadurch gekennzeichnet, daß man als Base Tetrahydroxyäthyl-ammonium-hydroxid der Formel

$$N(CH_2-CH_2-OH)_4^{\oplus}\ OH^{\ominus}$$

verwendet.

Europäisches Patentamt

# EUROPÄISCHER RECHERCHENBERICHT

0000026

Nummer der Anmeldung

EP 78 10 0041

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | GB - A - 1 457 608 (ICI LTD.) * Seite 1, Zeilen 21-28, 54-72; Seite 2, Zeilen 16-33; Seite 3, Zeilen 18-37 * | 1-5 |

KLASSIFIKATION DER ANMELDUNG (Int.Cl.²)

C 07 C 85/08

RECHERCHIERTE SACHGEBIETE (Int. Cl.²)

C 07 C 85/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 07-09-1978 | PAUWELS |